# EUROPEAN PATENT APPLICATION

(11) **EP 3 553 735 A1**
(43) Date of publication of application: **16.10.2019**
(21) Application number: 17908106.2
(22) Date of filing: 02.05.2017
(51) Int. Cl.: G06Q 50/22

(54) **SECURITY SYSTEM AND NODE DEVICE USED IN SAME**

(71) Applicant: Sustainable Medicine, Inc., Chuo-ku Tokyo 103-0023 (JP)
(72) Inventor: UENO Taro, Tokyo 1030023 (JP); ICHIKAWA Daisuke, Tokyo 1030023 (JP); KASHIYAMA Makiko, Tokyo 1030023 (JP)
(74) Representative: Michalski Hüttermann & Partner Patentanwälte mbB
(86) International application number: PCT/JP2017/017234
(87) International publication number: WO 2018/203382

(57) **Abstract**

Each of a plurality of node devices 200 connected by a distributed network includes a program storage unit 201 that stores a processing program for generating case report data by processing clinical trial data, a program execution unit 22 that generates case report data from clinical trial data, and a storage controller 24 that performs a consensus building process for sharing case report data among all the plurality of node devices 200 and causes a data storage unit 202 to store the case report data only when consensus is made. By preparing the case report data by execution of the processing program, falsification can be prevented in a process of preparing a case report even when a check, etc. by a third party is not included. Further, by distributing and storing the case report data in the respective node devices 200 only when validity is proved by the consensus building process, it is possible to prevent the case report from being falsified.

## Description

### Technical Field

The present invention relates to a security system and a node device used in the same, and is particularly suitable for use in a security system for preventing falsification of data related to a clinical trial.

### Background Art

In general, a clinical trial is conducted to verify safety, efficacy, etc. of a drug, a medical device, a treatment method, etc. In the clinical trial, management is normally performed such that after a doctor obtains various clinical trial data related to a patient by measurement, an inquiry, etc., contents of the clinical trial data are analyzed, and a case report is prepared and sent to a regulator to undergo an examination.

Here, to prevent falsification of the prepared case report, in a current system, a third party organization (CRO: Contract Research Organization) participates in management, and humans visually check validity of the clinical trial data. In addition, to ensure validity of the case report, a third party other than a person obtaining the clinical trial data may prepare the case report.

However, a system in which a third party organization or a third party (hereinafter collectively referred to simply as a "third party") intervenes in management is inefficient. For this reason, there has been a desire for development of a secure clinical trial system that prevents falsification of the case report without intervention of the third party.

Note that, Patent Documents 1 and 2 disclose inventions related to systems aiming to prevent falsification of clinical record data into which medical data of patients have been input.

According to a security ensuring method described in Patent Document 1, when an electronic clinical record is newly prepared, or an addition, a change, or a deletion of contents of the clinical record occurs, the medical institution communicates with a trusted organization therefor. The trusted organization provides a date timestamp to the medical institution communicating therewith. Thereafter, the medical institution transmits electronic clinical record data including new preparation, change, addition, or deletion of data of the electronic clinical record and the timestamp given from the trusted organization to the trusted organization. The trusted organization periodically receives a fixed date from a notary public for a medium storing the time stamp and the data, and seals and stores the received fixed date.

An electronic clinical record recording system described in Patent Document 2 is designed for the purpose of suppressing falsification of an electronic clinical record without using a timestamp whose reliability is uncertain. In the electronic clinical record recording system described in Patent Document 2, it is determined whether an electronic clinical record sent from a user terminal and recorded in an electronic clinical record reception memory contains content of an electronic clinical record recorded in an electronic clinical record database for storage, and the electronic clinical record of the electronic clinical record database for storage is overwritten only in a case in which it is determined that the whole content is contained.

Patent Document 1: JP-A-10-320491
Patent Document 2: JP-A-2011-103055

### Disclosure of the Invention

In the method described in Patent Document 1, the third part corresponding to the trusted organization needs to be involved, and a request to prevent falsification of the case report may not be fulfilled without intervention of the third party. Meanwhile, according to the system described in Patent Document 2, falsification of the electronic clinical record can be prevented without intervention of the third party. However, in the system described in Patent Document 2, even though falsification of the electronic clinical record can be prevented, it is impossible to prevent falsification of a case report prepared from medical data input to the electronic clinical record.

The invention has been made to solve such problems, and an object of the invention is to allow prevention of falsification of a product prepared from clinical trial data without intervention of a third party.

To solve the above-mentioned problems, in the invention, in a security system including a transmission terminal that transmits clinical trial data and a plurality of node devices connected by a distributed network, each of the plurality of node devices stores a processing program for generating a product of a processing result by processing the clinical trial data, generates the product by performing a process according to the processing program on the clinical trial data transmitted from the transmission terminal, and stores the product in a data storage unit. In this instance, a consensus building process for sharing the product among all the plurality of node devices is performed, and the product is stored in the data storage unit only when consensus is made.

According to the invention configured as described above, since preparation of the product from the clinical trial data is performed by execution of the processing program stored in the node device, even when a check, etc. by a third party is not included, falsification can be prevented in a process of preparing the product from the clinical trial data. In addition, the product prepared by execution of the processing program is distributed and stored in each node device only when validity is proved by a consensus building process among the plurality of node devices. Thus, for example, it is possible to prevent the product from being falsified by a malicious program installed in the node device. In this way, according to the invention, it is possible to suppress falsification of the product prepared from the clinical trial data without intervention of the third party.

### Brief Description of the Drawings

Fig. 1 is a diagram illustrating an overall configuration example of a security system according to first and second embodiments.
Fig. 2 is a block diagram illustrating a functional configuration example of a node device according to the first embodiment.
Fig. 3 is a flowchart illustrating an operation example of the node device according to the first embodiment.
Fig. 4 is a block diagram illustrating a functional configuration example of a node device according to the second embodiment.
Fig. 5 is a flowchart illustrating an operation example of the node device according to the second embodiment.
Fig. 6 is a diagram illustrating an overall configuration example of a security system according to a third embodiment.
Fig. 7 is a block diagram illustrating a functional configuration example of a node device according to the third embodiment.
Fig. 8 is a flowchart illustrating an operation example of the node device according to the third embodiment.

### Best Mode for Carrying Out the Invention

### (First embodiment)

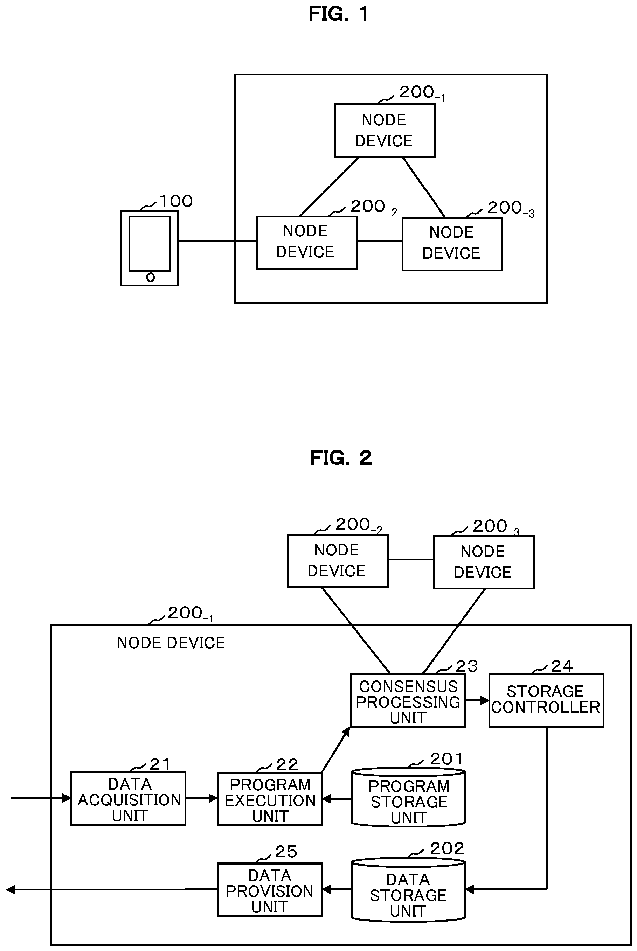

Hereinafter, an embodiment of the invention will be described with reference to drawings. Fig. 1 is a diagram illustrating an overall configuration example of a security system according to a first embodiment. As illustrated in Fig. 1, the security system according to the first embodiment includes a transmission terminal 100 that transmits clinical trial data and a plurality of node devices 200₋₁ to 200₋₃ connected by a distributed network (hereinafter may be collectively referred to as a node device 200).

The transmission terminal 100 may be a terminal used by a doctor at a medical institution or a terminal used by a patient undergoing a clinical trial. The transmission terminal 100 and the node device 200 are configured to be connectable by a communication network such as the Internet. Even though Fig. 1 illustrates a state in which the transmission terminal 100 is connected to the node device 200₋₂, the transmission terminal 100 may be arbitrarily connected to any of the node devices 200₋₁ to 200₋₃ in the distributed network.

The clinical trial data transmitted from the transmission terminal 100 includes measurement data measured using a medical device and inquiry data obtained by answering an inquiry. When the terminal of the patient is used as the transmission terminal 100, the inquiry data can be directly transmitted from the terminal of the patient. That is, it is possible to answer the inquiry at the terminal of the patient and transmit resultant inquiry data from the terminal of the patient. As for measurement data, after measurement is performed at the medical institution, the patient inputs the measurement data to the terminal of the patient, and transmits the measurement data from the terminal of the patient.

A block chain technology is introduced to the plurality of node devices 200₋₁ to 200₋₃ connected by the distributed network. That is, as described later, data is shared among the plurality of node devices 200₋₁ to 200₋₃ by the block chain technology. Note that, only three node devices 200₋₁ to 200₋₃ are illustrated in Fig. 1 for simplification of illustration, the number may be more than three.

Fig. 2 is a block diagram illustrating a functional configuration example of the node device 200 according to the first embodiment. Note that, here, even though a functional configuration of the node device 200₋₁ is illustrated, the other node devices 200₋₂ and 200₋₃ are similarly configured.

As illustrated in Fig. 2, as the functional configuration, the node device 200 according to the first embodiment includes a data acquisition unit 21, a program execution unit 22, a consensus processing unit 23, a storage controller 24, and a data provision unit 25. In addition, the node device 200 further includes a program storage unit 201 and a data storage unit 202 as storage media.

Each of the functional blocks 21 to 25 can be configured by any of hardware, a digital signal processor (DSP), and software. For example, in the case of being configured by software, each of the functional blocks 21 to 25 actually includes a central processing unit (CPU), a random access memory (RAM), a read only memory (ROM), etc., of a computer, and is realized by operating a program stored in a storage medium such as the RAM, the ROM, a hard disk, or a semiconductor memory.

The program storage unit 201 stores a processing program for processing clinical trial data and generating a product of a processing result. Content of the processing performed by the processing program on the clinical trial data can be arbitrarily determined. For example, the program may be set to a program that executes a process of analyzing the content of clinical trial data and preparing a case report to be submitted to a regulator. In this case, the product of the processing result obtained by execution of the processing program corresponds to data of the case report.

The data acquisition unit 21 acquires the clinical trial data transmitted from the transmission terminal 100. The program execution unit 22 performs the process according to the processing program stored in the program storage unit 201 on the clinical trial data acquired by the data acquisition unit 21 to generate the above-mentioned product (for example, case report data).

The consensus processing unit 23 performs a consensus building process for sharing the case report data, which is a product of execution of the processing program, among all the plurality of node devices 200₋₁ to 200₋₃. That is, the consensus processing unit 23 of the node device 200₋₁ transmits the case report data generated by the program execution unit 22 to the other node devices 200₋₂ and 200₋₃, and performs a predetermined consensus building process.

As the consensus building process performed by the consensus processing unit 23, it is possible to use a consensus algorithm known in the block chain technology. For example, the consensus processing unit 23 verifies validity of the case report data generated by the program execution unit 22 by performing the consensus building process using a consensus algorithm of practical byzantine fault tolerance (PBFT).

The storage controller 24 causes the data storage unit 202 to store the case report data that is the product only when the consensus processing unit 23 makes consensus. In this way, the case report data for which consensus is made is distributed and stored in the plurality of node devices 200₋₁ to 200₋₃.

The data provision unit 25 provides the case report data stored in the data storage unit 202 to an external terminal (not illustrated). For example, when a terminal of the regulator who examines clinical trial content based on the case report is used as the external terminal, the case report data can be provided to the terminal of the regulator. Here, to prevent data falsification during transmission, it is preferable to connect between the node device 200 and the terminal of the regulator by a dedicated line or to construct a virtual private network (VPN).

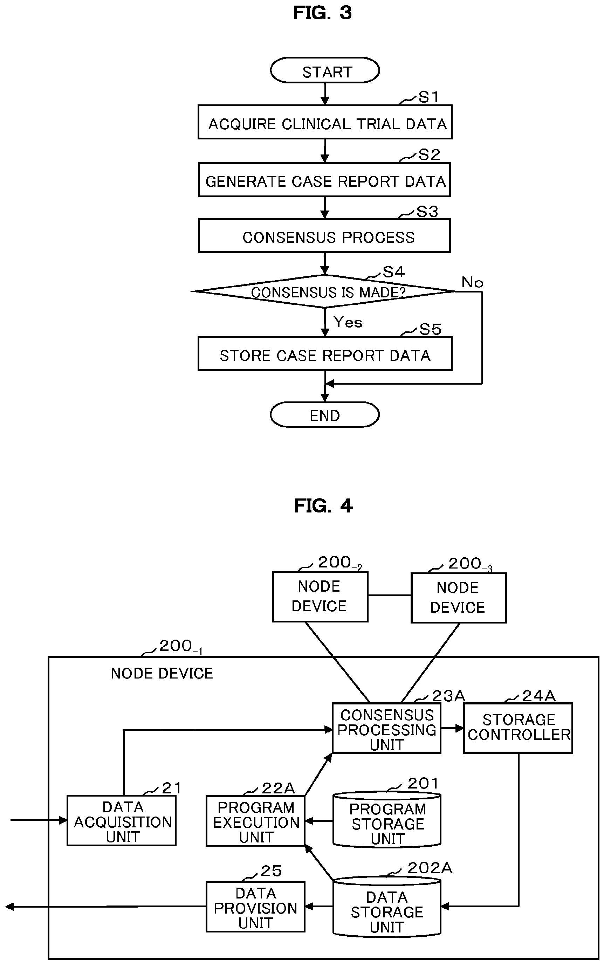

Fig. 3 is a flowchart illustrating an operation example of the node device 200 according to the first embodiment configured as described above.

First, the data acquisition unit 21 acquires clinical trial data transmitted from the transmission terminal 100 (step S1). When the data acquisition unit 21 acquires the clinical trial data, the program execution unit 22 generates case report data by processing the clinical trial data according to the processing program stored in the program storage unit 201 (step S2) .

Subsequently, the consensus processing unit 23 performs a consensus building process for sharing the case report data among all the plurality of node devices 200₋₁ to 200₋₃, and verifies validity of the case report data (step S3).

Here, the storage controller 24 determines whether consensus is made for the case report data by the consensus processing unit 23 (step S4). When consensus is successfully made for the case report data, the storage controller 24 stores the case report data in the data storage unit 202 (step S5). On the other hand, when consensus fails, the process of step S5 is not performed, and processing of the node device 200 ends.

As described above in detail, in the first embodiment, a processing program for processing clinical trial data to generate a product of a processing result is stored in each of the plurality of node devices 200₋₁ to 200₋₃. Then, case report data corresponding to a product is generated by processing the clinical trial data transmitted from the transmission terminal 100 to one node device 200₋₁ according to the processing program, and the case report data is stored in the data storage unit 202. In this instance, a consensus building process for sharing the generated case report data among all the plurality of node devices 200₋₁ to 200₋₃ is performed, and the case report data is stored in the data storage unit 202 only when consensus is made.

According to the first embodiment configured as described above, preparation of the case report data from the clinical trial data is not performed by an acquirer of the clinical trial data and is performed by execution of the processing program stored in the node device 200. For this reason, even when a check by the third party is not included, falsification can be prevented in a process of preparing the case report data from the clinical trial data.

In addition, the case report data prepared by execution of the processing program is distributed and stored in each of the node devices 200₋₁ to 200₋₃ only when the case report data is proved to be valid by the consensus building process among the plurality of node devices 200₋₁ to 200₋₃. For this reason, for example, it is possible to prevent the case report data from being falsified by a malicious program installed in the node device 200. In this way, according to the first embodiment, it is possible to suppress falsification of the product prepared from the clinical trial data without intervention of the third party.

### (Second embodiment)

Next, a second embodiment of the invention will be described with reference to drawings. An overall configuration example of a security system according to the second embodiment is similar to that of Fig. 1. Fig. 4 is a block diagram illustrating a functional configuration example of a node device 200 according to the second embodiment. Note that, in Fig. 4, a component to which the same reference numeral as a reference numeral illustrated in Fig. 2 has the same function, and thus a redundant description will be omitted here.

As illustrated in Fig. 4, the node device 200 according to the second embodiment includes a program execution unit 22A, a consensus processing unit 23A, a storage controller 24A, and a data storage unit 202A instead of the program execution unit 22, the consensus processing unit 23, the storage controller 24, and the data storage unit 202.

The data storage unit 202A stores clinical trial data acquired by the data acquisition unit 21 in addition to a product (case report data) obtained by execution of a processing program. However, as described below, the clinical trial data is stored in the data storage unit 202A only when consensus is successfully made for the clinical trial data among the plurality of node devices 200₋₁ to 200₋₃.

The consensus processing unit 23A performs each of a consensus building process for sharing the clinical trial data acquired by the data acquisition unit 21 among all the plurality of node devices 200₋₁ to 200₋₃ and a consensus building process for sharing case report data generated by the program execution unit 22A.

Here, the program execution unit 22A generates case report data corresponding to a product by performing a process according to a processing program stored in the program storage unit 201 on clinical trial data proved to be valid by the consensus building process, that is, the clinical trial data stored in the data storage unit 202A.

The storage controller 24A performs a control operation to cause the data storage unit 202 to store the clinical trial data and the case report data only when consensus is made by the consensus processing unit 23A. In this way, the clinical trial data for which consensus is made and case report data are distributed and stored in the plurality of node devices 200₋₁ to 200₋₃.

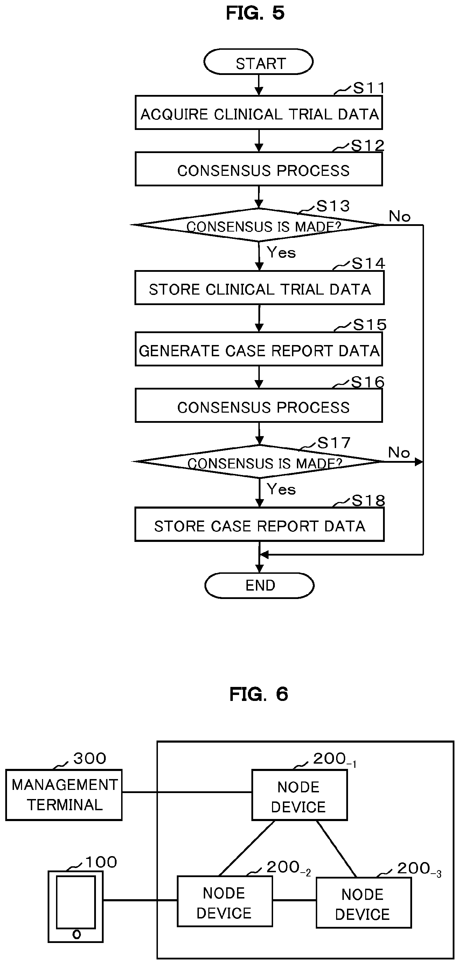

Fig. 5 is a flowchart illustrating an operation example of the node device 200 according to the second embodiment.

First, the data acquisition unit 21 acquires clinical trial data transmitted from the transmission terminal 100 (step S11). When the data acquisition unit 21 acquires the clinical trial data, the consensus processing unit 23A performs a consensus building process for sharing the clinical trial data among all the plurality of node devices 200₋₁ to 200₋₃, and verifies validity of the clinical trial data (step S12).

Here, the storage controller 24A determines whether consensus is made for the clinical trial data by the consensus processing unit 23A (step S13). When consensus is successfully made on the clinical trial data, the storage controller 24A stores the clinical trial data in the data storage unit 202A (step S14) . On the other hand, when consensus fails, processing of the node device 200 ends.

When consensus is successfully made for the clinical trial data, and the clinical trial data is stored in the data storage unit 202A, the program execution unit 22A generates case report data by performing a process according to a processing program stored in the program storage unit 201 on the clinical trial data stored in the data storage unit 202 (step S15).

Subsequently, the consensus processing unit 23A performs a consensus building process for sharing the case report data among all the plurality of node devices 200₋₁ to 200₋₃, and verifies validity of the case report data (step S16).

Here, the storage controller 24A determines whether consensus is made for the case report data by the consensus processing unit 23A (step S17). When consensus is successfully made for the case report data, the storage controller 24A causes the data storage unit 202A to store the case report data (step S18) . On the other hand, when consensus fails, the process of step S18 is not performed, and processing of the node device 200 ends.

According to the second embodiment configured as described above, in addition to the case report data corresponding to the product of execution of the processing program, the clinical trial data used for execution of the processing program is stored in the data storage unit 202A only when the clinical trial data is proved to be valid by the consensus building process. In this way, when it is necessary to submit not only the case report data but also the clinical trial data to the regulator, it is possible to suppress falsification of the clinical trial data.

In addition, in the second embodiment, processing of the processing program is executed using the clinical trial data proved to be valid by the consensus building process . Further, the consensus building process is performed on the generated case report data. In this way, validity of the case report data corresponding to the product is more secured. Therefore, according to the second embodiment, it is possible to more firmly suppress falsification of the product prepared from the clinical trial data without intervention of the third party.

### (Third embodiment)

Next, a third embodiment of the invention will be described with reference to the third embodiment. Fig. 6 is a diagram illustrating an overall configuration example of a security system according to the third embodiment. As illustrated in Fig. 6, the security system according to the third embodiment further includes a management terminal 300.

The management terminal 300 is a terminal used by a business operator, etc. who constructs and manages the security system. Similar to the transmission terminal 100, the management terminal 300 can be arbitrarily connected to any of the node devices 200₋₁ to 200₋₃ in the distributed network. The management terminal 300 transmits a processing program for performing predetermined processing on clinical trial data to a connected node device 200.

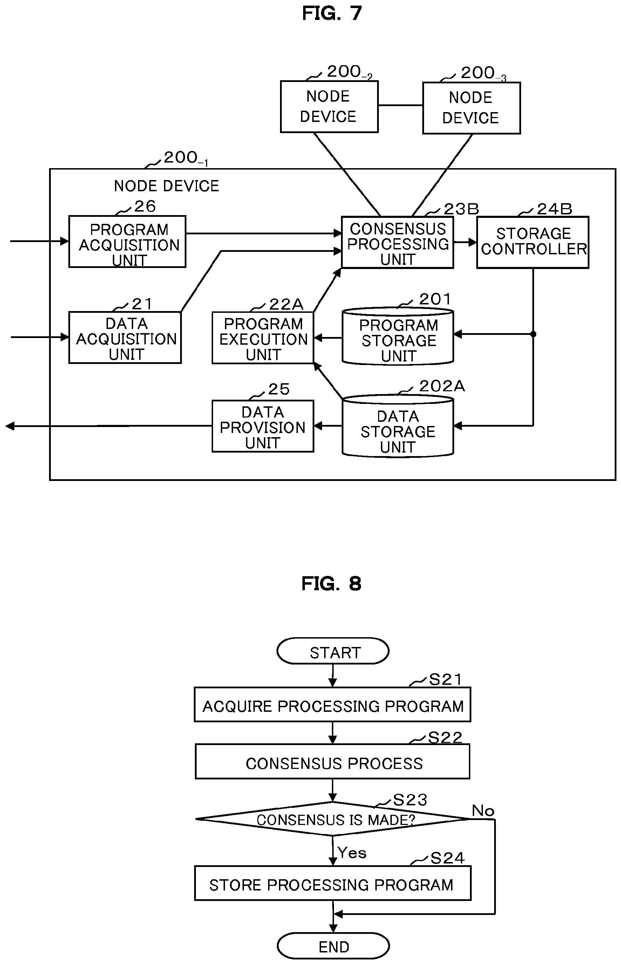

Fig. 7 is a block diagram illustrating a functional configuration example of the node device 200 according to the third embodiment. Note that, in Fig. 7, a component to which the same reference numeral as a reference numeral illustrated in Fig. 4 has the same function, and thus a redundant description will be omitted here.

As illustrated in Fig. 7, the node device 200 according to the third embodiment further includes a program acquisition unit 26. In addition, the node device 200 according to the third embodiment includes a consensus processing unit 23B and a storage controller 24B instead of the consensus processing unit 23A and the storage controller 24A.

The program acquisition unit 26 receives a processing program for generating a product of a processing result by processing clinical trial data from the management terminal 300.

The consensus processing unit 23B performs the same processing as that of the consensus processing unit 23A described in the second embodiment. That is, the consensus processing unit 23B performs each of a consensus building process for sharing clinical trial data acquired by the data acquisition unit 21 among all the plurality of node devices 200₋₁ to 200₋₃ and a consensus building process for sharing case report data generated by the program execution unit 22A.

Additionally, the consensus processing unit 23B performs a consensus building process for sharing a processing program acquired by the program acquisition unit 26 among all the plurality of node devices 200₋₁ to 200₋₃. That is, the consensus processing unit 23B transmits a processing program acquired by, for example, the program acquisition unit 26 of the node device 200₋₁ to the other node devices 200₋₂ and 200₋₃, and performs a predetermined consensus building process, thereby verifying validity of the processing program acquired by the program acquisition unit 26.

The storage controller 24B performs similar processing to that of the storage controller 24A described in the second embodiment. That is, the storage controller 24B performs a control operation to cause the data storage unit 202A to store the clinical trial data and the case report data only when consensus is made by the consensus processing unit 23B.

Additionally, the storage controller 24B performs a control operation to cause the program storage unit 201 to store the processing program only when consensus is made by the consensus processing unit 23B. In this way, the processing program for which consensus is made is distributed and stored in the plurality of node devices 200₋₁ to 200₋₃.

Fig. 8 is a flowchart illustrating an operation example of the node device 200 according to the third embodiment configured as described above. Note that, the flowchart illustrated in Fig. 8 illustrates a process at the time of registering a processing program in each node device 200. A process at the time of generating case report data using the registered processing program is similar to that of Fig. 5.

First, the program acquisition unit 26 acquires a processing program transmitted from the management terminal 300 (step S21). When the program acquisition unit 26 acquires the processing program, the consensus processing unit 23B performs a consensus building process for sharing the processing program among all the plurality of node devices 200₋₁ to 200₋₃, and verifies validity of the processing program (step S22) .

Here, the storage controller 24B determines whether consensus is made for the processing program by the consensus processing unit 23B (step S23). When consensus is successfully made for the processing program, the storage controller 24B causes the program storage unit 201 to store the processing program (step S24). On the other, when consensus fails, the process of step S24 is not performed, and processing of the node device 200 ends.

According to the third embodiment configured as described above, in addition to the clinical trial data and the case report data, the processing program for generating a case report from the clinical trial data is stored in the program storage unit 201 only when the processing program is proved to be valid by the consensus building process. In this way, since processing is executed using the processing program proved to be valid, validity of the product is more secured. Therefore, according to the third embodiment, it is possible to more firmly suppress falsification of the product prepared from the clinical trial data without intervention of the third party.

Note that, in the first to third embodiments, a description has been given of an example of adopting a program for executing a process of preparing a case report by analyzing content of clinical trial data as an example of the processing program. However, the invention is not limited thereto. For example, it is possible to adopt a processing program for preparing a clinical trial implementation plan describing an implementation plan of a future clinical trial by analyzing content of clinical trial data. Alternatively, it is possible to adopt a processing program for preparing a treatment policy manual describing a treatment policy for patients by analyzing content of clinical trial data.

In addition, in the first to third embodiments, a description has been given of an example of using PBFT as an example of the consensus algorithm. However, the invention is not limited thereto. For example, it is possible to use other consensus algorithms such as Proof of Work, Proof of Stake, Paxos, Raft, and Sieve.

In addition, any of the first to third embodiments is merely an example of embodiment for implementing the invention, and the technical scope of the invention should not be interpreted in a limited manner by these embodiments. That is, the invention can be implemented in various forms without departing from the scope or main features of the invention.

### Reference Signs List

- 21: Data acquisition unit
- 22, 22A: Program execution unit
- 23, 23A, 23B: Consensus processing unit
- 24, 24A, 24B: Storage controller
- 25: Data provision unit
- 26: Program acquisition unit
- 100: Transmission terminal
- 200: Node device
- 201: Program storage unit
- 202, 202A: Data storage unit

## Claims

1. A security system comprising:
a transmission terminal that transmits clinical trial data; and
a plurality of node devices connected by a distributed network,
each of the plurality of node devices includes
a program storage unit that stores a processing program for generating a product of a processing result by processing the clinical trial data,
a program execution unit that generates the product by performing a process according to the processing program stored in the program storage unit on the clinical trial data transmitted from the transmission terminal,
a data storage unit that stores the product generated by the program execution unit,
a consensus processing unit that performs a consensus building process for sharing the product among all the plurality of node devices, and
a storage controller that performs a control operation to cause the data storage unit to store the product only when consensus is made by the consensus processing unit, and
the product for which consensus is made is distributed and stored in the plurality of node devices.

2. The security system according to claim 1,
the data storage unit stores the clinical trial data transmitted from the transmission terminal in addition to the product,
the consensus processing unit performs each of a consensus building process for sharing the clinical trial data and a consensus building process for sharing the product among all the plurality of node devices,
the storage controller performs a control operation to cause the data storage unit to store the clinical trial data and the product only when consensus is made by the consensus processing unit, and
the clinical trial data and product for which consensus is made are distributed and stored in the plurality of node devices.

3. The security system according to claim 2, the program execution unit performs a process according to the processing program stored in the program storage unit on clinical trial data proved to be valid by the consensus building process by the consensus processing unit.

4. The security system according to any one of claims 1 to 3,
the consensus processing unit further performs a consensus building process for sharing the processing program among all the plurality of node devices, and
the storage controller performs a control operation to cause the program storage unit to store the processing program only when consensus is made by the consensus processing unit.

5. A node device connected by a distributed network, comprising:
a program storage unit that stores a processing program for generating a product of a processing result by processing clinical trial data;
a program execution unit that generates the product by performing a process according to the processing program stored in the program storage unit on the clinical trial data transmitted from a transmission terminal;
a data storage unit that stores the product generated by the program execution unit;
a consensus processing unit that performs a consensus building process for sharing the product among all of a plurality of node devices connected to the distributed network; and
a storage controller that performs a control operation to cause the data storage unit to store the product only when consensus is made by the consensus processing unit.
